# EUROPEAN PATENT APPLICATION

(11) **EP 2 008 652 A1**
(43) Date of publication of application: **31.12.2008**
(21) Application number: 07111335.1
(22) Date of filing: 28.06.2007
(51) Int. Cl.: A61K 31/343, A61P 35/00

(54) **Griseofulvin analogues for the treatment of cancer by inhibition of centrosomal clustering**

(71) Applicant: Deutsches Krebsforschungszentrum Stiftung des öffentlichen Rechts, 69120 Heidelberg (DE)
(72) Inventor: Krämer, Alwin, 76149 Karlsruhe (DE); Rebacz, Blanka, 69123 Heidelberg (DE); Larsen, Thomas O., 2480 Holte (DK); Clausen, Mads Hartvig, 2200 Kopenhagen N (DK)
(74) Representative: Huhn, Michael

(57) **Abstract**

The present invention relates to uses of compounds having a structure as shown by formula (1) for the manufacture of a pharmaceutical composition for the treatment of cancer. Moreover, the present invention encompasses methods of treatment for said diseases.

## Description

The present invention relates to uses of compounds having a structure as shown by formula (I) for the manufacture of a pharmaceutical composition for the treatment of cancer. The present invention encompasses methods of treatment for such diseases. The present invention is also directed to a part of the compounds disclosed in the present application as such.

A drawback of the current standard therapy for cancer using surgery, chemotherapy, high dosage chemotherapy including stem cell transplantation, radiation and ¹³¹I-MIBG therapy is the limited efficacy and selectivity accompanied by varying lethality rates, as the case may be. Moreover, serious side effects are regularly encountered.

Classical anti-cancer drugs use proliferation/cell division as a target, thereby killing all dividing cells without differentiating between cells belonging to the tumour that should be targeted and normal tissues. This lack of specificity causes most of the well-known and unavoidable side effects of classical chemotherapeutical agents.

There is a long-felt need to provide or identify compounds, which can be used for the treatment of cancer, the anti-tumoral mode of action of which shall not be based on the cytotoxic principles of traditional chemotherapy and which are selective for cancer cells only, not for normal, non-transformed cells of the body.

Griseofulvin (Chemical Abstract name: (2-5-trans)-7-chloro-2,4,6-trimethoxy-6-methylspiro-[benzofuran-2(3H), 1-(2)-cyclohexene]-3,4-dione; Chem. Abstr. Ser. No. 126-07-8; chemical structure see structure (II)) is a natural antibiotic produced by *Penicillium griseofulvum* as well as other microfungi and was isolated in 1938.

Griseofulvin is still commonly used in humans for the treatment of dermatomycoses in skin, hair, and nails caused by *Microsporum, Trichophyton,* and *Epidermophyton.* The mode of action on fungi is not fully understood, but it has been shown that it causes a reversible block of protein and nucleic acid synthesis and that its main effect on mitosis is apparently due to disorganization of the spindle microtubules. The daily dose for adults is 0.5 to 1 g (maximal 20 mg/kg bw.); in children it is 10 mg/kg bw. The treatment time depends on the type and localization of the infection, for hair infections 2 to 3 months, for onychomycoses and nail infections approximately 6 months are required. Griseofulvin is also used in veterinary medicine against ringworm *(Trichophyton)* infections.

Patent application WO 97/05870 discloses the use of griseofulvin for inhibiting the growth of cancers, particularly in humans. The compound can be used to inhibit the growth of leukemia, tumors and cancer cells. The disclosure of this application is restricted to griseofulvin alone.

Oda discloses in J. Antibiot. 59(2), pages 114-116 (2006) that an analogue of griseofulvin in which the methoxy group in 2' position has been replaced by a n-propoxy group exhibits a stronger inhibiting effect on cancer cells than griseofulvin itself.

The inhibiting effect of griseofulvin is however not regarded as being sufficient to allow its use in the treatment of cancer.

It is, therefore, an object of the present invention to provide methods for treating cancer, the anti-tumoral mode of action of which shall not be based on the cytotoxic principles of traditional chemotherapy because this will not improve the results of the therapy. A further object of the present invention is the provision of compounds which are active in the treatment of cancer and which are not based on the cytotoxic traditional chemotherapy principles. Furthermore, only cancer cells shall be effected but not "normal" non-transformed cells of the body. In particular, the compounds shall have better properties for the treatment of cancer than griseofulvin or its anti-cancer activity tested analogues, with respect to their anti-tumor activity as well as with respect to the spectrum of cancers which can be treated.

This object is attained by a compound according to the general formula (I)

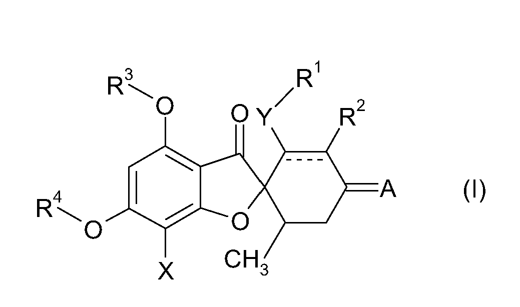

In the most general meaning classified below under A, the symbols have the following meanings:

### A:

A is selected from: =O, =NOR⁵, and =N-NR⁶R⁷, and =NR⁸;
X is selected from: H, halogen and pseudohalogen;
Y is selected from: -CH₂-, -O-, -S- and -NH-;
wherein the dotted line denotes a double bond which may optionally be present;
R¹ is selected from: acyclic and cyclic, linear and branched alkyl, alkenyl and alkynyl groups having 4 to 20 carbon atoms, wherein the named groups can be unsubstituted or substituted or carry one or more substituents from the group halogen, pseudohalogen, -C(O)OH, -NO₂, NH₂, -OH and -O(C₁-C₄)alkyl; linear or branched acyclic alkyl groups having 1 to 20 carbon atoms and carrying in their chain one or more non-adjacent atoms from the group O and S; and aralkyl groups having a linear or branched acyclic alkyl group with 1 to 20 carbon atoms and an aromatic group having 5 to 10 carbon atoms, in which the aromatic group optionally contains one or more heteroatoms selected from O, S or N in its cycle;
R² is selected from: H; acyclic and cyclic, linear and branched alkyl, alkenyl and alkynyl groups having 1 to 20 carbon atoms, wherein the named groups can be unsubstituted or substituted or carry one or more substituents from the group halogen, pseudohalogen, -C(O)OH, -NO₂, NH₂, -OH and -O(C₁-C₄)alkyl; linear or branched acyclic alkyl groups having 1 to 20 carbon atoms and carrying in their chain one or more non-adjacent atoms from the group O and S; and aralkyl groups having a linear or branched acyclic alkyl group with 1 to 20 carbon atoms and an aromatic group having 5 to 10 carbon atoms, in which the aromatic group optionally contains one or more heteroatoms selected from O, S or N in its cycle;
R³ is selected from: H; acyclic and cyclic, linear and branched alkyl, alkenyl and alkynyl groups having 1 to 10 carbon atoms; and aralkyl groups having a linear or branched acyclic alkyl group with 1 to 10 carbon atoms and an aromatic group having 5 or 6 carbon atoms;
R⁴ independently has the same meaning as R³ ;
R⁵ is H or a linear or branched alkyl group having 1 to 4 carbon atoms or an acyl group having 1 to 4 carbon atoms;
R⁶ is H or a linear or branched alkyl group having 1 to 4 carbon atoms;
R⁷ independently has the same meaning as R⁶;
R⁸ independently has the same meaning as R⁶;
or a pharmaceutically acceptable salt thereof, for the manufacture of a pharmaceutical composition for the treatment of cancer.

In the context of the present invention, an aralkyl group is a group based on an alkyl group by which an aromatic group is linked to the rest of the molecule to which it is attached. A prominent example of an aralkyl group is the benzyl group -CH₂-(C₆H₅), wherein -CH₂- is linked to the rest of the molecule and furthermore carries a phenyl group -(C₆H₅).

The double bonds in the non-aromatic ring of the compounds of formula (I) may give rise to tautomerism from e.g. an enol/keto equilibrium. In case tautomers result from the presence of the exocyclic double bond, a given tautomeric form may be stabilised or fixed by alkylation. Suitable alkylation methods are known to the person skilled in the art.

The present invention also includes prodrugs of the compounds of formula (I).

### Preferred embodiments are classified below under B:

### B:

A is selected from: =O, =NOR⁵;
X is selected from: H, halogen and pseudohalogen;
Y is selected from: -CH₂-, -O-;
wherein the dotted line denotes a double bond which may optionally be present;
R¹ is selected from: acyclic and cyclic, linear and branched alkyl, alkenyl and alkynyl groups having 4 to 15 carbon atoms, wherein the named groups can be unsubstituted or substituted or carry one or more substituents from the group halogen, pseudohalogen, -C(O)OH, -NO₂, NH₂, -OH and -O(C₁-C₄)alkyl; linear or branched alkylenoxy groups having 1 to 20 carbon atoms; and aralkyl groups having a linear or branched acyclic alkyl group with 1 to 10 carbon atoms and an aromatic group having 5 or 6 carbon atoms, in which the aromatic group optionally contains one or more heteroatoms selected from O, S or N in its cycle;
R² is selected from: H; acyclic and cyclic, linear and branched alkyl, alkenyl and alkynyl groups having 1 to 15 carbon atoms, wherein the named groups can be unsubstituted or substituted or carry one or more substituents from the group halogen, pseudohalogen, -C(O)OH, -NO₂, NH₂, -OH and -O(C₁-C₄)alkyl; linear or branched alkylenoxy groups having 1 to 20 carbon atoms; and aralkyl groups having a linear or branched acyclic alkyl group with 1 to 10 carbon atoms and an aromatic group having 5 or 6 carbon atoms, in which the aromatic group optionally contains one or more heteroatoms selected from O, S or N in its cycle;
R³ is selected from: H; acyclic and cyclic, linear and branched alkyl, alkenyl and alkynyl groups having 1 to 6 carbon atoms; and aralkyl groups having a linear or branched acyclic alkyl group with 1 to 6 carbon atoms and an aromatic group having 5 or 6 carbon atoms;
R⁴ independently has the same meaning as R³ ;
R⁵ is H or a linear or branched alkyl group having 1 to 4 carbon atoms or an acyl group having 1 to 4 carbon atoms;
or a pharmaceutically acceptable salt thereof, for the manufacture of a pharmaceutical composition for the treatment of cancer.

### Even more preferred embodiments are classified below under C:

### C:

A is selected from: =O, =NOR⁵;
X is selected from: H, halogen and pseudohalogen;
Y is selected from: -CH₂-, -O-;
wherein the dotted line denotes a double bond which may optionally be present;
R¹ is selected from: acyclic and cyclic, linear and branched alkyl, alkenyl and alkynyl groups having 4 to 10 carbon atoms, wherein the named groups can be unsubstituted or substituted or carry one or more substituents from the group halogen, pseudohalogen, -C(O)OH, -OH and -O(C₁-C₄)alkyl; ethylenoxy groups having 1 to 20 carbon atoms and propylenoxy groups having 1 to 20 carbon atoms; and aralkyl groups having a linear or branched acyclic alkyl group with 1 to 6 carbon atoms and an aromatic group having 5 or 6 carbon atoms, in which the aromatic group optionally contains one or more heteroatoms selected from O, S or N in its cycle;
R² is selected from: H; acyclic and cyclic, linear and branched alkyl, alkenyl and alkynyl groups having 1 to 10 carbon atoms, wherein the named groups can be unsubstituted or substituted or carry one or more substituents from the group halogen, pseudohalogen, -C(O)OH, -OH and -O(C₁-C₄)alkyl; ethylenoxy groups having 1 to 20 carbon atoms and propylenoxy groups having 1 to 20 carbon atoms; and aralkyl groups having a linear or branched acyclic alkyl group with 1 to 6 carbon atoms and an aromatic group having 5 or 6 carbon atoms, in which the aromatic group optionally contains one or more heteroatoms selected from O, S or N in its cycle;
R³ is selected from: H; acyclic and cyclic, linear and branched alkyl, alkenyl and alkynyl groups having 1 to 6 carbon atoms; and aralkyl groups having a linear or branched acyclic alkyl group with 1 to 6 carbon atoms and an aromatic group having 5 or 6 carbon atoms;
R⁴ independently has the same meaning as R³ ;
R⁵ is H or a linear or branched alkyl group having 1 to 4 carbon atoms or an acyl group having 1 to 4 carbon atoms;
or a pharmaceutically acceptable salt thereof, for the manufacture of a pharmaceutical composition for the treatment of cancer.

### Embodiments, which are still preferred over those under C, are classified below under D:

### D

A is selected from: =O, =NOR⁵;
X is selected from: H, halogen;
Y is selected from: -O-;
wherein the dotted line denotes a double bond which may optionally be present;
R¹ is selected from: acyclic and cyclic, linear and branchched alkyl, alkenyl and alkynyl groups having 5 to 8 carbon atoms, wherein the named groups can be unsubstituted or substituted or carry one or more substituents from the group halogen, pseudohalogen, -C(O)OH, -OH and -O(C₁-C₄)alkyl; ethylenoxy groups having 1 to 10 carbon atoms and propylenoxy groups having 1 to 10 carbon atoms; and aralkyl groups having a linear or branched acyclic alkyl group with 1 to 4 carbon atoms and an aromatic group having 5 or 6 carbon atoms, in which the aromatic group optionally contains one or more heteroatoms selected from O, S or N in its cycle;
R² is selected from: H; acyclic and cyclic, linear and branchched alkyl, alkenyl and alkynyl groups having 1 to 8 carbon atoms, wherein the named groups can be unsubstituted or substituted or carry one or more substituents from the group halogen, pseudohalogen, -C(O)OH, -OH and -O(C₁-C₄)alkyl; ethylenoxy groups having 1 to 10 carbon atoms and propylenoxy groups having 1 to 10 carbon atoms; and aralkyl groups having a linear or branched acyclic alkyl group with 1 to 4 carbon atoms and an aromatic group having 5 or 6 carbon atoms, in which the aromatic group optionally contains one or more heteroatoms selected from O, S or N in its cycle;
R³ is selected from: H; acyclic and cyclic, linear and branched alkyl, alkenyl and alkynyl groups having 1 to 4 carbon atoms; and aralkyl groups having a linear or branched acyclic alkyl group with 1 to 4 carbon atoms and an aromatic group having 5 or 6 carbon atoms;
R⁴ independently has the same meaning as R³ ;
R⁵ is H or a linear or branched alkyl group having 1 to 4 carbon atoms or an acyl group having 1 to 4 carbon atoms;
or a pharmaceutically acceptable salt thereof, for the manufacture of a pharmaceutical composition for the treatment of cancer.

Within the definitions made beforehand for embodiments A, B, C and D, the individual meanings for each substituent or each group are not construed to be restricted to the embodiment under which it is mentioned, i.e. a definition cited under C may be combined with the definitions made under e.g. A, B, or D. The entire combination of all the definitions for each substituent made under each of A, B, C and D is however preferred.

As an example, for the best known compound close to the formula (I) and which is not included in the present invention is griseofulvin according to the formula (II)

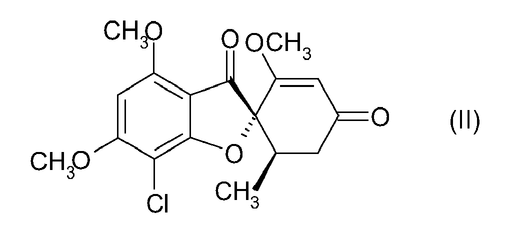

The cancer varieties which can be treated with the compounds as defined beforehand are human malignancies, preferably solid neoplasias or haematological malignancies. Examples for such malignancies comprise brain cancer, head- and neck cancer, renal cancer, breast cancer, esophageal cancer, gastric cancer, colon cancer, liver cancer, lung cancer, pancreas cancer, biliary tract cancer, prostate cancer, skin cancer, melanoma, ovarian cancer, cervical cancer, sarcoma, bone and soft tissue sarcomas, leukemia, multiple myeloma and lymphoma, including both Hodgkin and Non-Hodgkin lymphomas.

Basically, all human malignancies are potential targets for centrosomal cluster inhibitors/griseofulvin/griseofulvin analogues since almost all malignant neoplasias examined to date harbour centrosome aberrations. Specifically, centrosome aberrations have been reported in solid tumors of different origin including brain, breast, lung, cervical colon, pancreatic, biliary tract, prostate, and head and neck cancers. Also, sarcomas and hematological malignancies including Hodgkin and Non-Hodgkin lymphomas, acute and chronic myeloid leukemias, chronic lymphocytic leukemias and multiple myelomas have been described to harbour centrosomal abnormalities. Importantly, since several preneoplastic lesions like, cervical intraepithelial neoplasias, ductal carcinoma in situ of the mammary gland, colon and pancreatic adenomas, pre-invasive carcinomas in situ of the prostate, myelodysplastic syndromes, and monoclonal gammopathies of undetermined significance contain centrosome aberrations as well, the above therapy might also serve to prevent progression of these lesions into invasive carcinomas, leukaemia or multiple myeloma, respectively.

In a further embodiment, the present invention relates to a compound according to formula (I) as defined above in general form or in preferred embodiments, or a pharmaceutically acceptable salt thereof, for use as medicament.

The term "pharmaceutical composition" is sometimes referred to as "pharmaceutical" or "medicament" hereinafter or in the prior art. Said terms shall have the same meaning and may be used interchangeably.

The compounds according to the formula (I) act as an inhibitor of centrosome clustering. They force tumor cells with supernumerary centrosomes to undergo multipolar mitoses and, subsequently, apoptosis.

Moreover, the compounds are specific for the tumours because they elicit no effects on healthy body cells with a normal centrosome content. Accordingly, there are no or only minor side effects to be expected.

Centrosomes are small cytoplasmic organelles which consist of a pair of centrioles embedded in pericentriolar material and act as microtubule organizing centers. During mitosis, centrosomes function as spindle poles, directing the formation of bipolar spindles, a process essential for accurate chromosome segregation. Since the centrosome duplicates precisely once per cell cycle, each daughter cell receives one centrosome upon cytokinesis and contains two centrosomes at the time of mitosis.

Centrosome amplification has been frequently observed in both solid tumors and hematological malignancies and is linked to tumorigenesis and aneuploidy. The extent of centrosomal aberrations is correlated with the degree of chromosomal instability and clinical aggressiveness of the malignant neoplasias. In mitosis, supernumerary centrosomes can lead to the formation of multipolar spindles which are responsible for chromosome malsegregation with subsequent aneuploidy and which can be found in many tumor types. Multipolar spindles, however, are antagonistic to cell viability. Most progeny derived from a defective mitosis will undergo apoptosis, but few daughter cells, receiving the appropriate chromosome complement and gene dosage, may survive and contribute, via clonal selection, to a population of aneuploid tumor cells. The survivors, however, must overcome the condition of supernumerary centrosomes in order to divide efficiently. To regain secondary karyotype stability, many tumor cells have developed a mechanism termed centrosomal clustering that prevents the formation of multipolar spindles by coalescence of multiple centrosomes into two functional spindle poles.

Centrosome positioning in the center of interphase cells is accomplished by pulling forces applied to microtubules by dynein, which serves to keep the centrosome away from the cell margin, and microtubule pushing by actomyosin-driven forces directed toward the cell center. Several pieces of evidence suggest that the minus-end-directed microtubule motor protein dynein is involved in microtubule minus end bundling for the establishment of bipolar spindles. A current model suggests that the function of dynein to tether spindle pole microtubules into bundles requires NuMA, which might use the motor activity of dynein to become localized to centrosomes. At the spindle poles, it forms a matrix to hold microtubule minus ends together. Analogous, in cells with multiple centrosomes, centrosomal clustering seems to be mediated by dynein. Recent data show that only cells with spindle-associated dynein localization were capable of coalescing multiple centrosomes into two spindle poles. Spindle multipolarity, on the other hand, was found to follow overexpression of NuMA which interferes with the spindle localization of dynein. With the exception of the involvement of dynein and NuMA, the molecular mechanisms responsible for clustering of multiple centrosomes into two spindle poles of tumor cells are unknown.

The only known small molecules that specifically affect the mitotic machinery target either tubulin or the plus-end-directed motor protein Eg5, a mitotic kinesin required for spindle bipolarity. Whereas vinca alkaloids and taxanes disrupt spindle function by inhibiting or increasing microtubule polymerization, inhibition of Eg5 activity by monastrol leads to impaired microtubule-dependent centrosome separation and formation of monopolar spindles. Both vinca alkaloids and taxanes are used as anticancer drugs and Eg5 is currently evaluated as a potential target for antineoplastic drug development. However, neither microtubule poisoning nor Eg5 inhibition selectively affects tumor cells, explaining side effects and dose limitations of antimitotic drugs in clinical use.

Supernumerary centrosomes do almost exclusively occur in a wide variety of neoplastic disorders but not in non-transformed cells. Therefore, inhibition of centrosomal clustering with consequential induction of multipolar spindles and subsequent cell death would specifically target tumor cells with no impact on normal cells with a regular centrosome content. To identify cell-permeable small molecules that inhibit centrosomal clustering in cells with supernumerary centrosomes, we developed a cell-based screening strategy founded on the visualization of microtubules and chromatin.

Natural products have proved to be rich sources of novel anti-cancer lead compounds during the past 20 years. Therefore, we decided to screen a fungal extract library for compounds inhibiting centrosomal clustering. The fungal extracts were selected based on a chemotaxonomic screening approach, in order to increase the chemodiversity to be tested. An initial screening effort using extracts from different *Penicillium* species led to the identification of griseofulvin as an inhibitor of centrosome coalescence in several different tumor cell lines.

The concentrations of the compounds used according to the present invention necessary for the induction of multipolar spindles are similar to those required for the inhibition of mitosis and cell proliferation, suggesting that multipolar spindles lead to aberrant cell divisions and subsequent cell death. The cytotoxicity induced by the compounds used according to the present invention is limited to cells with multipolar spindles. Unlike those, cells with bipolar spindles, despite experiencing a prolonged mitosis, eventually divide and survive in the presence of the compounds of the invention.

The compounds having a structure as shown in the general formula (I) are more potent than the known compound griseofulvin.

Specifically, growth, cell cycle and viability of "normal" non-transformed body cells is not affected by the compounds of the invention.

A part of the compounds of the general formula (I) are known as such. These known compounds do not belong to the present invention, only with respect to its particular pharmacological and biological properties, as laid out beforehand. The compounds according to the formula (I) which are not known (i.e. new and/or inventive over the prior art) are a part of the present invention.

The present invention includes the use of all stereoisomeric forms of the compounds of the formula (I) for the purposes laid out herein, i.e. for the treatment of cancer, particular the cancer varieties which can be treated with a compound of the formula (I). The present invention also includes, for the compounds as defined beforehand which are not known, all stereoisomeric forms of these compounds. Centers of asymmetry that are present in the compounds of formula (I) all independently of one another have S configuration or R configuration. The invention includes all possible enantiomers and diastereomers and mixtures of two or more stereoisomers, for example mixtures of enantiomers and/or diastereomers, in all ratios. Thus, compounds according to the present invention which can exist as enantiomers can be present in enantiomerically pure form, both as levorotatory and as dextrorotatory antipodes, in the form of racemates and in the form of mixtures of the two enantiomers in all ratios. In the case of a cis/trans isomerism the invention includes both the cis form and the trans form as well as mixtures of these forms in all ratios. All these forms are an object of the present invention. The preparation of individual stereoisomers can be carried out, if desired, by separation of a mixture by customary methods, for example by chromatography or crystallization, by the use of stereochemically uniform starting materials for the synthesis or by stereoselective synthesis. Optionally a derivatization can be carried out before a separation of stereoisomers. The separation of a mixture of stereoisomers can be carried out at the stage of the compounds of the formula (I) or at the stage of an intermediate during the synthesis. The present invention also includes all tautomeric forms of the compounds of formula (I).

In case the compounds according to formula (I) contain one or more acidic or basic groups, the invention also comprises their corresponding pharmaceutically or toxicologically acceptable salts, in particular their pharmaceutically utilizable salts. Thus, the compounds of the formula (I), which contain acidic groups, can be present on these groups and can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I), which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) simultaneously contain acidic and basic groups in the molecule, the invention also includes, in addition to the salt forms mentioned, inner salts or betaines (zwitterions). The respective salts according to the formula (I) can be obtained by customary methods, which are known to the person skilled in the art like, for example by contacting these with an organic or inorganic acid or base in a solvent or dispersant, or by anion exchange or cation exchange with other salts. The present invention also includes all salts of the compounds of the formula (I), which, owing to low physiological compatibility, are not directly suitable for use in pharmaceuticals but which can be used, for example, as intermediates for chemical reactions or for the preparation of pharmaceutically acceptable salts.

The present invention furthermore includes all solvates of compounds of the formula (I), for example hydrates or adducts with alcohols, active metabolites of the compounds of the formula (I), and also derivatives and prodrugs of the compounds of the formula (I) which contain physiologically tolerable and cleavable groups, for example esters, amides and compounds in which the N-H group depicted in formula (I) is replaced with an N-alkyl group, such as N-methyl, or with an N-acyl group, such as N-acetyl or N-argininyl, including pharmaceutically acceptable salts formed on functional groups present in the N-acyl group.

The compounds according to general formula (I) and their precursors can be prepared according to methods published in the literature or, respectively, analogous methods. Appropriate methods have been published in, for example, Houben-Weyl, Methoden der Organischen Chemie (Methods of Organic Chemistry), Thieme-Verlag, Stuttgart, or Organic Reactions, John Wiley & Sons, New York.

All reactions for the synthesis of the compounds of the formula (I) are per se well-known to the skilled person and can be carried out under standard conditions according to or analogously to procedures described in the literature, for example in Houben-Weyl, Methoden der Organischen Chemie (Methods of Organic Chemistry), Thieme-Verlag, Stuttgart, or Organic Reactions, John Wiley & Sons, New York. Depending on the circumstances of the individual case, in order to avoid side reactions during the synthesis of a compound of the formula (I), it can be necessary or advantageous to temporarily block functional groups by introducing protective groups and to deprotect them in a later stage of the synthesis, or introduce functional groups in the form of precursor groups which in a later reaction step are converted into the desired functional groups. Such synthesis strategies and protective groups and precursor groups, which are suitable in an individual case, are known to the skilled person. If desired, the compounds of the formula (I) can be purified by customary purification procedures, for example by recrystallization or chromatography. The starting compounds for the preparation of the compounds of the formula (I) are commercially available or can be prepared according to or analogously to literature procedures. The compounds obtained with the above-mentioned synthesis methods are a further object of the present invention.

The compounds according to the formula (I) can also be used in combination with other pharmaceutically active compounds, preferably compounds which are able to enhance the effect of the compounds according to the general formula (I). Examples of such compounds include: (i) antimetabolites, cytarabine, fludarabine, 5-fluoro-2'-deoxyuridine, gemcitabine, hydroxyurea or methotrexate; (ii) DNA-fragmenting agents, bleomycin, (iii) DNA-crosslinking and alkylating agents, chlorambucil, cisplatin, carboplatin, fotemustine, cyclophosphamide, ifosfamide, dacarbazine or nitrogen mustard; (iv) intercalating agents, adriamycin (doxorubicin) or mitoxantrone; (v) protein synthesis inhibitors, L-asparaginase, cycloheximide, puromycin or diphteria toxin; (vi) topoisomerase I poisons, camptothecin or topotecan; (vii) topoisomerase II poisons, etoposide (VP-16) or teniposide; (viii) microtubule-directed agents, colcemid, colchicine, paclitaxel (taxol), docetaxel (taxotere), vinblastine or vincristine; (ix) kinase inhibitors, flavopiridol, staurosporin, STI571 (CPG 57148B) or UCN-01 (7-hydroxystaurosporine); (x) miscellaneous investigational agents, trichostatin A, thioplatin, PS-341, phenylbutyrate, ET-18-OCH₃, or farnesyl transferase inhibitors (L-739749, L-744832); polyphenols, quercetin, resveratrol, piceatannol, epigallocatechine gallate, theaflavins, flavanols, procyanidins, betulinic acid and derivatives thereof; (xi) hormones, glucocorticoids or fenretinide; (xii) hormone antagonists, tamoxifen, finasteride or LHRH antagonists, (xiii) demethylating agents, 5-azacytidine, 5-aza-2'deoxycytidine, 5,6-dihydro-5-azacytidine, or (xiv) a combination of any of the pharmaceuticals given above or use in high-dose chemotherapy regimens including stem cell transplantation; (xv) differentiation inducing agents such as retinoic acid derivatives; (xvi) ionizing radiation therapy, MIBG-therapy and conventional radiation therapy.

The compounds of the formula (I) and their pharmaceutically acceptable salts, optionally in combination with other pharmaceutically active compounds, can be administered to animals, preferably to mammals, and in particular to humans, as pharmaceuticals by themselves, in mixtures with one another or in the form of pharmaceutical preparations. Further subjects of the present invention therefore also are the compounds of the formula (I) and their pharmaceutically acceptable salts for use as pharmaceuticals, including the use of the pharmaceuticals as inhibitor of centrosome clustering, to induce multipolar mitoses of tumor cells with supernumerary centrosomes, and to induce apoptosis. They can be used in the therapy and prophylaxis of the above-mentioned diseases and syndromes as well as for preparing pharmaceuticals for these purposes. Furthermore, subjects of the present invention are pharmaceutical preparations (or pharmaceutical compositions), which comprise an effective dose of at least one compound of the formula (I) and/or a pharmaceutically acceptable salt thereof and a pharmaceutically acceptable carrier, i.e. one or more pharmaceutically acceptable carrier substances and/or additives.

The pharmaceuticals according to the invention can be administered orally, for example in the form of pills, tablets, lacquered tablets, sugar-coated tablets, granules, hard and soft gelatin capsules, aqueous, alcoholic or oily solutions, syrups, emulsions or suspensions, or rectally, for example in the form of suppositories. Administration can also be carried out parenterally, for example subcutaneously, intramuscularly or intravenously in the form of solutions for injection or infusion. Other suitable administration forms are, for example, percutaneous or topical administration, for example in the form of ointments, tinctures, sprays or transdermal therapeutic systems, or the inhalative administration in the form of nasal sprays or aerosol mixtures, or, for example, microcapsules, implants or rods. The preferred administration form depends, for example, on the disease to be treated and on its severity.

The preparation of the pharmaceutical preparations can be carried out in a manner known per se. To this end, one or more compounds of the formula (I) and/or their pharmaceutically acceptable salts, together with one or more solid or liquid pharmaceutical carrier substances and/or additives (or auxiliary substances) and, if desired, in combination with other pharmaceutically active compounds having therapeutic or prophylactic action, are brought into a suitable administration form or dosage form, which can then be used as a pharmaceutical in human or veterinary medicine.

For the production of pills, tablets, sugar-coated tablets and hard gelatin capsules it is possible to use, for example, lactose, starch, for example maize starch, or starch derivatives, talc, stearic acid or its salts, etc. Carriers for soft gelatin capsules and suppositories are, for example, fats, waxes, semisolid and liquid polyols, natural or hardened oils, etc. Suitable carriers for the preparation of solutions, for example of solutions for injection, or of emulsions or syrups are, for example, water, physiologically sodium chloride solution, alcohols such as ethanol, glycerol, polyols, sucrose, invert sugar, glucose, mannitol, vegetable oils, etc. It is also possible to lyophilize the compounds of the formula (I) and their pharmaceutically acceptable salts and to use the resulting lyophilisates, for example, for preparing preparations for injection or infusion. Suitable carriers for microcapsules, implants or rods are, for example, copolymers of glycolic acid and lactic acid.

Besides the compound or compounds according to the invention and carriers, the pharmaceutical preparations can also contain additives, for example fillers, disintegrants, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents or antioxidants.

The dosage of the compound of the formula (I) to be administered and/or of a pharmaceutically acceptable salt thereof depends on the individual case and is, as is customary, to be adapted to the individual circumstances to achieve an optimum effect. Thus, it depends on the nature and the severity of the disorder to be treated, and also on the sex, age, weight and individual responsiveness of the human or animal to be treated, on the efficacy and duration of action of the compounds used, on whether the therapy is acute or chronic or prophylactic, or on whether other active compounds are administered in addition to compounds of the formula (I). The daily dose can be administered in a single dose or, in particular when larger amounts are administered, be divided into several, for example two, three or four individual doses. In some cases, depending on the individual response, it may be necessary to deviate upwards or downwards from the given daily dose.

It is to be understood that the compounds identified in accordance with the method of the present invention may be used as pharmaceutical compositions to treat cancer and, preferably, even the cancer varieties referred to beforehand. Accordingly, the method of the present invention may further comprise the steps of manufacturing the identified compound as a pharmaceutical composition as described elsewhere in this specification.

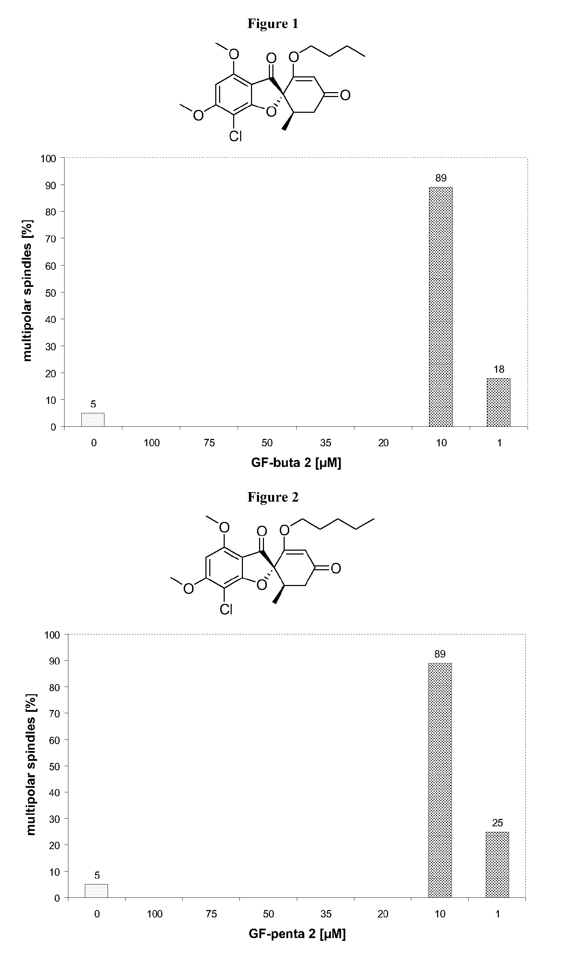

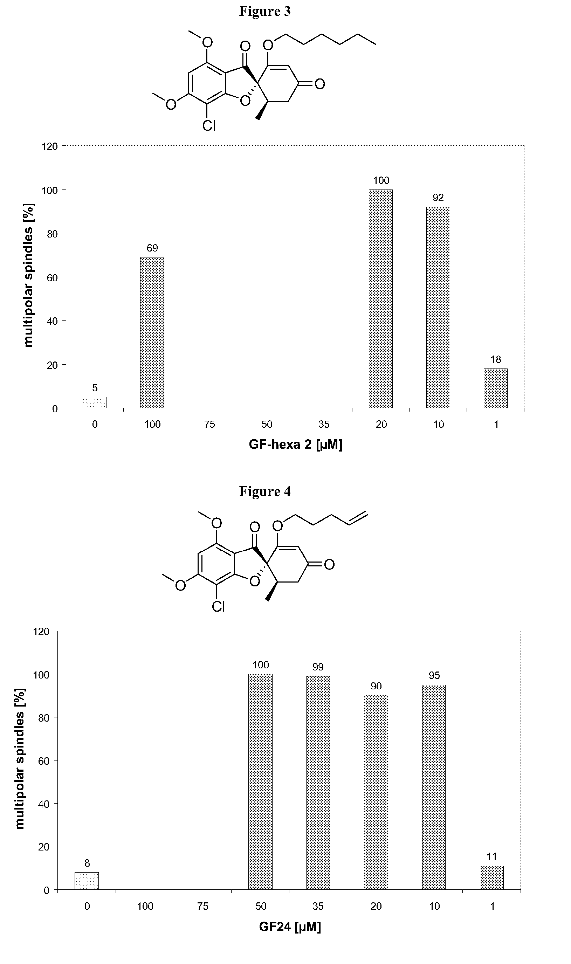

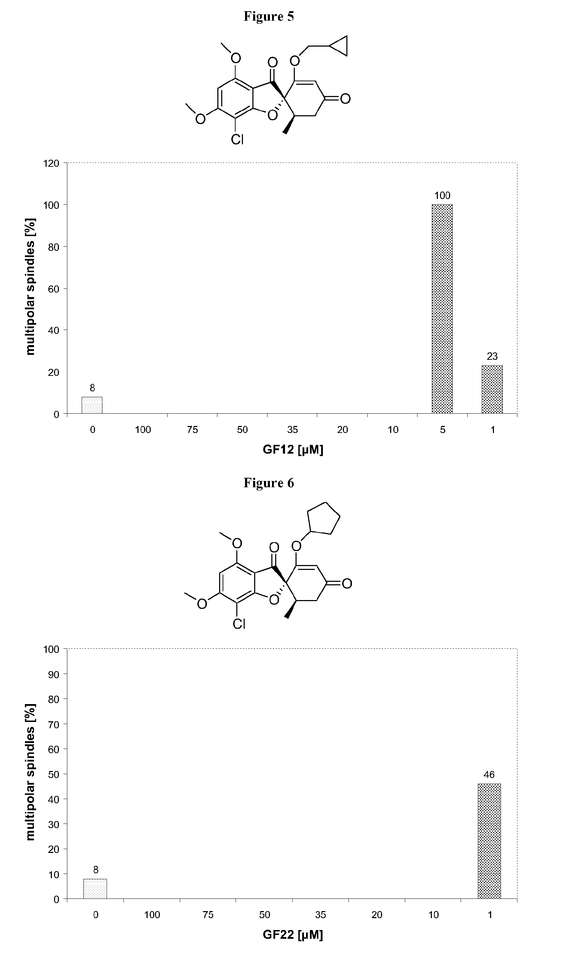

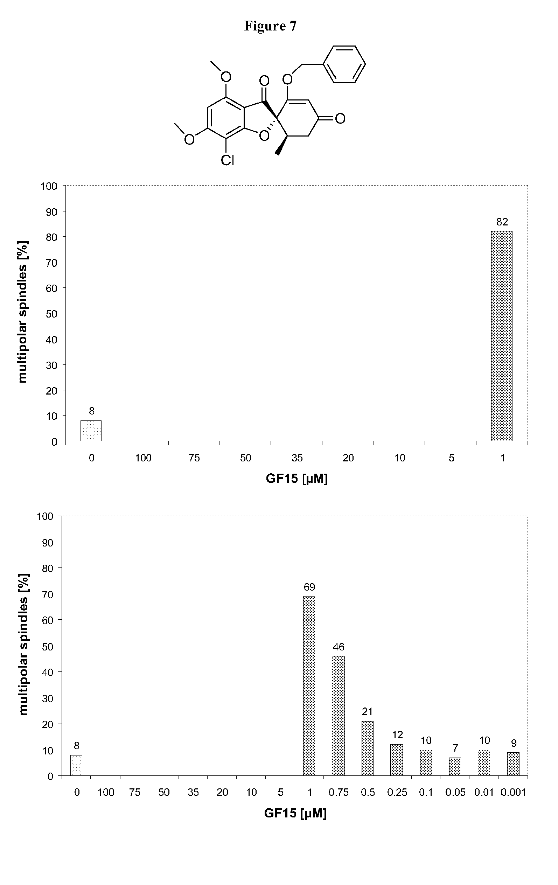

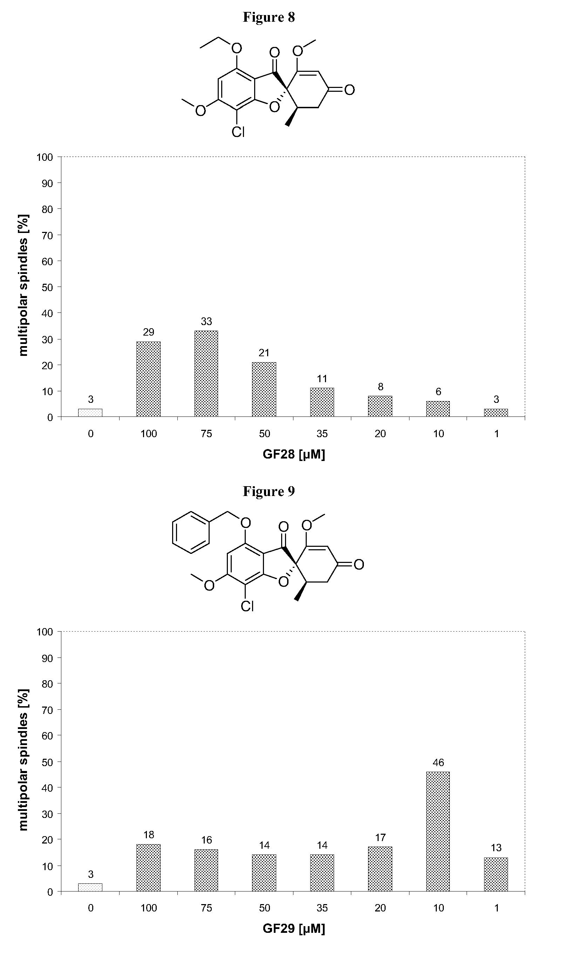

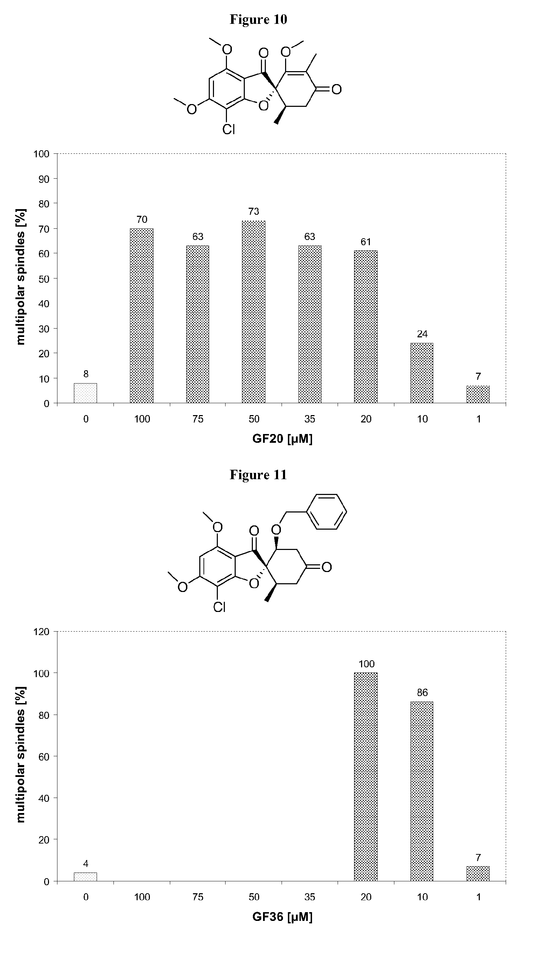

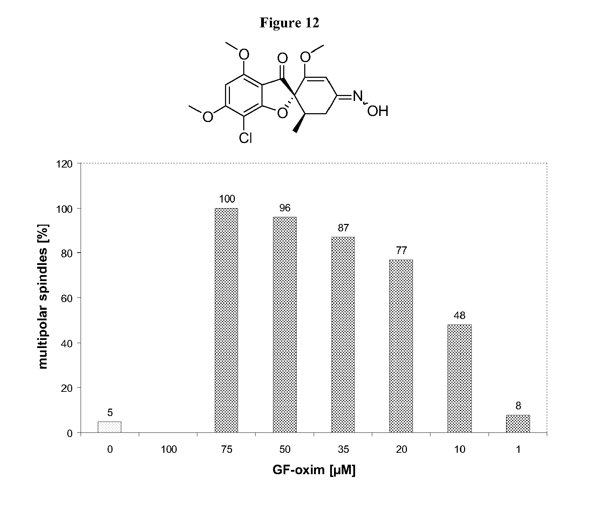

The figures 1 to 12 give representative examples of compounds, which have been prepared in the framework of the present invention, together with their activity, which has been determined as described below. The graphs show the percentage of cells exhibiting multipolar mitotic spindles. For example, a number of 65% would mean that 65% of all mitotic cells, are exhibit multipolar spindles and 35% exhibit bipolar spindles.

The invention will now be illustrated by the following Examples. However, the Examples are not meant to limit the scope of the invention in any respect.

### Materials and Methods

**Cell Culture.** All cell lines were cultured in Dulbecco's modified Eagle's Medium (DMEM; PAA Laboratories, Pasching, Austria) supplemented with 10 % FCS (PAA). SCC114 cells stably expressing GFP-α-tubulin were generated by transfection (Fugene 6, Roche Diagnostics, Mannheim, Germany) of the transgene in pEGFP-C1 (Clontech, Heidelberg, Germany) and maintained under selective pressure by addition of geniticin (Invitrogen, Karlsruhe, Germany). Primary normal human epidermal keratinocytes (NHEK; PromoCell, Heidelberg, Germany) were cultured in Keratinocyte Growth Medium 2 (PromoCell). When indicated, griseofulvin (Sigma, Deisenhofen, Germany) or a griseofulvin analogue was added to the culture medium. Griseofulvin or the griseofulvin analogue was dissolved in DMSO (Sigma). In all experiments, the final DMSO concentration was 0.1 %.

**Antibodies.** The following antibodies were used: mouse monoclonal antibodies to Eg5 (Transduction Laboratories, Lexington, KY), α-tubulin (DM1A), γ-tubulin (GTU-88) (Sigma, Deisenhofen, Germany), δ-tubulin (A1), ε-tubulin (H280), PARP (F-2) (Santa Cruz, Heidelberg, Germany) dynein light intermediate chain (Chemicon International, Hampshire, UK), and NuMA (Calbiochem, Darmstadt, Germany); rabbit polyclonal antibodies to γ-tubulin, centrin (Sigma), pericentrin (Covance, Richmond, CA), actin (I-19) (Santa Cruz), and phospho-S10-histone H3 (Upstate Biotechnology, Lake Placid, NY). A mouse monoclonal antibody to centrin and a rabbit polyclonal antibody to c-Nap1 was kindly provided by J.L. Salisbury, Rochester, MN and E.A. Nigg, Martinsried, Germany, respectively.

**Immunofluorescence.** Immunofluorescence staining was performed as described (Krämer et al., 2004). The following fluorochrome-conjugated secondary antibodies were used: anti-rabbit Alexa 488 (Molecular Probes, Invitrogen, Karlsruhe, Germany) and anti-mouse Cy3 (Jackson ImmunoResearch Laboratories, West Grove, PN). Immunostained cells were examined using a Zeiss Axiovert 200 M fluorescence microscope (Göttingen, Germany). Images were processed with Photoshop software (Adobe, Munich, Germany).
[Krämer A, Mailand N, Lukas C, et al. Centrosome-associated Chk1 prevents premature activation of cyclin-B-Cdk1 kinase. Nat Cell Biol 2004;6:884-891]

**Time-lapse video microscopy.** For live-cell imaging, GFP-α-tubulin expressing SCC114 cells were grown in CO₂-independent Leibovitz's medium (Gibco, Invitrogen, Karlsruhe, Germany) on plastic dishes (µ-dishes, Ibidi, Munich, Germany). Live-cell imaging was carried out using a Nikon TE2000-U inverted microscope equipped with differential interference contrast optics and an Orca AG camera (Hamamatsu), driven by NIS-Element AR software (Nikon). Individual GFP-α-tubulin expressing cells containing bipolar or multipolar spindles were detected by immunofluorescence and followed using differential interference contrast imaging.

**Flow cytometry.** Cell cycle analysis by flow cytometry including the quantification of cells in mitosis by phospho-S10-histone H3 staining was performed as previously described (Syljuasen et al., 2004)).
[Syljuasen RG, Sorensen CS, Nylandsted J, Lukas C, Lukas J, Bartek J. Inhibition of Chk1 by CEP-3891 accelerates mitotic nuclear fragmentation in response to ionizing Radiation. Cancer Res 2004;64:9035-40]

**Colorimetric MTT (tetrazolium) assay.** The cytotoxicity assay was performed as previously described (Mosmann, 1983).
[Mosmann T. Rapid colorimetric assay for cellular growth and survival: application to proliferation and cytotoxicity assays. J Immunol Methods 1983;65:55-63]

**Isolation and analysis of human centrosomes.** Centrosomes from SCC114 cells were isolated as previously described (Krämer et al., 2004; Blomberg-Wirschell and Doxsey, 1998).
[Krämer A, Mailand N, Lukas C, et al. Centrosome-associated Chk1 prevents premature activation of cyclin-B-Cdk1 kinase. Nat Cell Biol 2004;6:884-891]
[Blomberg-Wirschell M, Doxsey SJ. Rapid isolation of centrosomes. Methods Enzymol 1998;298:228-38]

**Measurement of Annexin-V-positive cells.** Phosphatidylserine externalization was analyzed using the Apoptosis Detection Kit I from Becton Dickinson (Heidelberg, Germany) according to the manufacturer's recommendations.

**Fungal strains examined.** All fungal isolates were obtained from the IBT Culture Collection at BioCentrum-DTU, Technical University of Denmark. The cultures were inoculated in triplicate on Czapek yeast (autolysate) agar (CYA) and yeast extract sucrose agar (YES) and incubated at 25 °C for 7 days in the dark. *Penicillium adametzii;* IBT 21918, 22617. *P*. alkaloidigenum; IBT 13424. *P*. arizonae; 12285, 12289. *P*. berlinense; IBT 4099, 18288, 19440. *P. bilaiae*; IBT 14448, 14960. *P. brasilianum*; IBT 13220, 22244. *P*. chilense; IBT 22472. *P*. coralligenum; IBT 5972. *P. decumbens;* IBT 11843, 24323. *P. digitatum*; IBT 5533, 23020. *P*. faroense, IBT 22543, 22955. *P. gerundense;* IBT 19242, 19244. *P*. graminiarum; IBT 22393. *P. implicatum;* IBT 19329, 22878. *P*. indicum; IBT 14840, 21937. *P. italicum;* IBT 21533, 23026. *P*. kubunicum; IBT 20435. *P*. *lapatayae;* IBT 10870, 23667. *P. lividum;* IBT 22614. *P*. neodonkii; IBT 13306, 16446. *P*. novodecumbens; IBT 22238, 22239. *P. palmense;* IBT 4912, 23009. *P*. stallonei; IBT 20688, 20718. *P. svalbardense;* IBT 23856, 23949.

**Preparation of fungal extracts.** Procedures for micro-extraction of fungal secondary metabolites were similar to those given by Smedsgaard (1997) with the following differences: Plug extracts were prepared from five plugs and extracted with 2 ml of EtOAc/CH₂Cl₂/MeOH (3:2:1) containing 50 ppm of trifluoroacetic acid. The extracts were taken to dryness using a rotary vacuum centrifugation system (RVC).
[Smedsgaard J. Micro-scale extraction procedure for standardized screening of fungal metabolite production in cultures. J Chromatography 1997;760:264-70]

**Treatment of GFP-α-tubulin expressing SCC114 cells with fungal extracts.** Starting material was (on average) 3.5 mg of each raw fungal extract. Each extract consisted of (on average) 10 major active compounds with a median molecular weight of 350 (200 - 500) Dalton. Since 1:200 to 1:10⁷ fold dilutions from a 3.5 mg/100 µl (10 mM per active compound) stock solution were used in our assay system, the resulting final concentrations per compound and extract ranged from nanomolar to micromolar.

**Fractionation of fungal extracts and isolation of pure compounds by semi-preparative HPLC.** Extracts positive for inhibition of centrosomal clustering were fractionated into 24 fractions (4 ml) to track the activity into single (or few) compounds on a Phenomenex Luna II C₁₈ column (250 mm x 10 mm, 5 µm). The column was run at room temperature with a flow rate of 4 ml/min starting with H₂O/CH₃CN at 85:15 going up to 0:100 in 20 min followed by isocratic at 100 % CH₃CN for 4 min. Solvents were HPLC-grade acetonitrile and destilled water, both with 50 ppm TFA added. All fractions were taken to dryness on a RVC. Positive fractions were further purified as described above however this time collecting single peaks in order to obtain very pure compounds such as griseo fulvin.

**Identification of griseofulvin by HPLC-DAD-MS.** Griseofulvin containing fractions were analyzed using an Agilent HP 1100 liquid chromatograph with a DAD system (Waldbronn, Germany) coupled to a LCT oaTOF mass spectrometer (Micromass, Manchester, UK) using a Z-spray ESI source and a Lock Spray probe according to Nielsen and Smedsgaard (2003). A Phenomenex Luna II C₁₈ column (100 mm x 2 mm, 3 µm) at 40°C and a flow rate of 0.3 ml/min was used for separation. Solvents were HPLC-grade acetonitrile and destilled water. The water and acetonitrile were buffered with 20 mM formic acid.
[Nielsen, K. F. and Smedsgaard, J. Fungal metabolite screening: database of 474 mycotoxins and fungal metabolites for dereplication by standardised liquid chromatography-UV-mass spectrometry methodology. Journal of chromatography, *1002*: 111-136, 2003]

**Synthesis of griseofulvin derivatives.** Griseofulvin and all other chemicals were purchased from Sigma-Aldrich and used without further purification. Thin-layer chromatography was performed on aluminum plates precoated with silica gel. Flash chromatography was performed using Merck silica gel 60. ¹H NMR spectra were recorded on a Varian Unity Inova 500 spectrometer and ¹³C NMR spectra on a Bruker AC 200 spectrometer operating at 50 MHz. IR spectra were obtained using a Perkin-Elmer 1600 FT-IR instrument. Melting points were determined using a Heidolph capillary melting point apparatus and are uncorrected. Microanalyses were conducted by H. Kolbe Mikroanalytisches Laboratorium, Mülheim an der Ruhr, Germany.

### Synthesis of representative, active compounds of the formula (I):

General procedure 1 for compounds with variation in R₁: To a solution of griseofulvin [Arkley et al., 1962] in the appropriate alcohol was added camphor sulfonic acid (0.15 equiv.). The mixture was stirred at 80-120 °C for 1-24 h, cooled to 20 °C and EtOAc was added to the solution. The mixture was washed with sat. aq. NaH₂PO₄ and then water. The combined aqueous phases were extracted with EtOAc and the combined organic phases were dried (MgSO₄) and concentrated. The residue was purified by column chromatography (EtOAc and heptane) to give the product and the regioisomer. Crystalline compounds were re-crystallized from EtOAc and heptane.

General procedure 2 for compounds with variation in R₁: To a solution of griseofulvic acid [Arkley et al., 1962] in anhydrous dioxane was added the appropriate alcohol (5 equiv.) and camphor sulfonic acid (0.15 equiv.). The mixture was stirred at 80 °C for 22 h and cooled to 20 °C. EtOAc was added to the solution and the mixture was washed with sat. aq. NaHCO₃. The aqueous phase was extracted with EtOAc and the organic layer was dried (MgSO₄) then concentrated. The residue was purified by column chromatography (EtOAc and heptane) to give the product and the regioisomer. Crystalline compounds were re-crystallized from EtOAc and heptane.

General procedure 3 for compounds with variation in R₃: To a solution of (2*S,*6*'R*)-(7-chloro-6-methoxy-4-hydroxy-benzofuran-3-on)-2-spiro-1'-(2'-methoxy-6'-methyl-cyclohex-2'-en-4'-one) [Arkley et al., 1962] in DMF was added K₂CO₃ (1 equiv.) and NaH (2 equiv.). After 20 min. the appropriate alkyl bromide (2 equiv.) was added. More alkyl bromide was added in intervals (5 equiv. per portion). After 30 h the solution was diluted with EtOAc and washed with sat. aq. NH₄Cl. The aqueous phase was extracted with EtOAc and the organic layer was dried (MgSO₄) then concentrated. The residue was purified by column chromatography (EtOAc and heptane) to give the product, which was re-crystallized from EtOAc and heptane.

The following representative compounds were prepared according to general procedure 1:
(2*S*,6'*R*)-(7-chloro-4,6-dimethoxy-benzofuran-3-on)-2-spiro-1'-(2'-butoxy-6'-methyl-cyclohex-3'-en-4'-one): m.p. 152-154°C; IR (KBr, cm⁻¹): 1706, 1662 (C=O), 1614 (C=C); ¹H-NMR (CDCl₃): δ 6.11 (1H, s), 5.50 (1H, s), 4.03 (3H, s), 3.97 (3H, s), 3.83-3.68 (2H, m), 3.03 (1H, dd, *J* = 16.5, 13.5 Hz), 2.79 (1H, ddq, *J* = 13.3, 6.6, 4.7 Hz), 2.41 (1H, dd), 1.59-1.49 (2H, m), 1.28-1.19 (2H, m), 0.95 (3H, d, *J* = 6.7 Hz), 0.74 (3H, t, *J* = 7.4 Hz); ¹³C-NMR (CDCl₃): δ 197.1, 192.9, 170.4, 169.5, 164.7, 157.9, 105.4, 105.2, 97.2, 91.1, 89.6, 69.5, 57.2, 56.6, 40.2, 36.5, 30.2, 19.0, 14.5, 13.8; Anal. Calcd for C₂₀H₂₃ClO₆: C, 60.84; H, 5.87, found: C, 60.71; H, 5.83.
(2*S*,6'*R*)-(7-chloro-4,6-dimethoxy-benzofuran-3-on)-2-spiro-1'-(2'-pentoxy-6'-methyl-cyclohex-3'-en-4'-one): m.p. 146-148 °C; IR (KBr, cm⁻¹): 1701, 1653 (C=O), 1613 (C=C); ¹H-NMR (CDCl₃): δ 6.11 (1H, s), 5.48 (1H, s), 4.02 (3H, s), 3.96 (3H, s), 3.79-3.66 (2H, m), 3.03 (1H, dd, *J* = 16.5, 13.5 Hz), 2.82 (1H, ddq, *J =* 13.5, 6.7, 4.6 Hz), 2.40 (1H, dd, *J* = 16.5, 4.6 Hz), 1.55 (2H, quintet, *J* = 6.5 Hz), 1.20-1.15 (2H, m), 0.95 (3H, d, *J* = 6.7 Hz), 0.77 (3H, t, *J* = 6.8 Hz); ¹³C-NMR (CDCl₃): δ 197.4, 192.8, 170.4, 169.9, 164.7, 157.9, 105.4, 105.1, 97.4, 91.1, 89.5, 69.8, 57.2, 56.6, 40.2, 36.4, 28.0, 27.9, 22.3, 14.5, 14.1; Anal. Calcd for C₂₁H₂₅ClO₆: C, 61.69; H, 6.16, found: C, 61.73; H, 5.99.
(2*S*,6'*R*)-(7-chloro-4,6-dimethoxy-benzofuran-3-on)-2-spiro-1'-(2'-hexoxy-6'-methyl-cyclohex-3'-en-4'-one): m.p. 152-154 °C; IR (KBr, cm⁻¹): 1701, 1663 (C=O), 1616 (C=C); ¹H-NMR (CDCl₃): δ 6.11 (1H, s), 5.50 (1H, s), 4.03 (3H, s), 3.97 (3H, s), 3.83-3.66 (2H, m), 3.04 (1H, dd, *J* = 16.5, 13.4 Hz), 2.83 (1H, ddq, *J* = 13.4, 6.6, 4.6 Hz), 2.41 (1H, dd, *J* = 16.5, 4.6 Hz), 1.55 (2H, quintet, *J* = 6.5 Hz), 1.25-1.16 (6H, m), 0.96 (3H, d, *J* = 6.7 Hz), 0.80 (3H, t, *J* = 6.9 Hz); ¹³C-NMR (CDCl₃): δ 192.8, 170.4, 169.8, 164.7, 157.9, 105.5, 105.1, 97.6, 91.1, 89.5, 69.7, 57.2, 56.6, 54.7, 40.2, 36.4, 31.3, 28.2, 25.4, 22.6, 14.5, 14.0; Anal. Calcd for C₂₂H₂₇ClO₆: C, 62.48; H, 6.44, found: C, 62.39; H, 6.37.
(2*S*,6'*R*)-(7-chloro-4,6-dimethoxy-benzofuran-3-on)-2-spiro-1'-(2'-pent-4-enyloxy-6'-methyl-cyclohex-2'-en-4'-one): IR (KBr, cm⁻¹): 1707, 1654 (C=O), 1613 (C=C); ¹H-NMR(CDCl₃): δ 6.13 (1H, s), 5.65 (1H, ddt, *J* = 17.2, 10.5, 6.8 Hz), 5.50 (1H, s), 4.93 (1H, dd, *J* = 17.2, 1.8 Hz), 4.90 (1H, dd, *J* = 10.5, 1.8 Hz), 4.03 (3H, s), 3.97 (3H, s), 3.81 (1H, dt, *J* = 9.6, 6.2 Hz), 3.73 (1H, dt, *J* = 9.6, 6.2 Hz), 3.04 (1H, dd, *J* = 16.7, 13.5 Hz), 2.82 (1H, ddt, *J* = 13.5, 4.7, 6.7 Hz), 2.41 (1H, dd, *J* = 16.7, 4.7 Hz), 2.00-1.94 (2H, m), 1.70-1.63 (2H, m), 1.02 (3H, d, *J* = 6.7 Hz); 13C-NMR(CDCl₃): δ 197.1, 192.5, 170.0, 169.5, 164.4, 157.6, 136.9, 115.6, 104.9 (2C), 97.0, 90.8, 89.3, 68.4, 56.9, 56.3, 39.9, 36.2, 29.6, 27.2 14.2; EIMS: m/z calcd for C₂₁H₂₃ClO₆ M+ 406, found 406.

The following representative compounds were prepared according to general procedure 2:
(2*S*,6'*R*)-(7-chloro-4,6-dimethoxy-benzofuran-3-on)-2-spiro-1'-(2'-cyclopropylmethoxy-6'-methyl-cyclohex-2'-en-4'-one): m.p.: 190-191 °C; IR (KBr, cm⁻1): 1704, 1659 (C=O), 1608 (C=C); ¹H-NMR(CDCl₃): δ 6.13 (1H, s), 5.47 (1H, s), 4.03 (3H, s), 3.98 (3H, s), 3.65 (2H, d, *J* = 6.5, Hz), 3.03 (1H, dd, *J* = 16.7, 13.5 Hz), 2.83 (1H, ddq., *J* = 13.5, 4.7, 6.6 Hz), 2.41 (1H, dd, *J* = 16.7, 4.7 Hz), 1.05-0.98 (1H, m), 0.96 (3H, d, *J* = 6.6 Hz), 0.50-0.43 (2H, m), 0.22-0.13 (2H, m); ¹³C-NMR(CDCl₃: δ 197.0, 192.5, 169.9, 169.6, 164.4, 157.6, 105.0 (2C), 97.1, 90.8, 89.3, 73.2, 56.9, 56.3, 39.9, 36.2, 14.2, 9.0, 2.7 (2C); Anal. Calcd for C₂₀H₂₁ClO₆: C, 61.15; H, 5.39, found: C, 60.95; H, 5.45.
(2*S*,6'*R*)-(7-chloro-4,6-dimethoxy-benzofuran-3-on)-2-spiro-1'-(2'-cyclopentoxy-6'-ethyl-cyclohex-2'-en-4'-one): IR (KBr, cm⁻¹): 1705, 1652 (C=O), 1615 (C=C); ¹H-NMR(CDCl₃): δ 6.11 (1H, s), 5.49 (1H, s), 4.56-4.51 (1H, m), 4.03 (3H, s), 3.97 (3H, s), 3.03 (1H, dd, *J* = 16.7, 13.5 Hz), 2.82 (1H, ddq, *J* = 13.5, 4.8, 6.7 Hz), 2.40 (1H, dd, *J* = 16.7, 4.8 Hz), 1.79-1.72 (2H, m), 1.72-1.64 (2H, m), 1.58-1.44 (4H, m), 0.95 (3H, d, *J* = 6.7 Hz), ¹³C-NMR(CDCl₃): δ 197.1, 192.6, 169.6, 169.0, 164.3, 157.5, 105.8, 105.1, 97.0, 90.9, 89.2, 81.6, 56.8, 56.3, 39.8, 36.1, 32.1 (2C), 23.7 (2C) 14.2; EIMS: m/z calcd for C₂₁H₂₃ClO₆ [M+] 406, found 406.
(2*S*,6'*R*)-(7-chloro-4,6-dimethoxy-benzofuran-3-on)-2-spiro-1'-(2'-benzyloxy-6'-methyl-cyclohex-2'-en-4'-one): m.p.: 186-188 °C; ¹H-NMR(CDCl₃): δ 7.32-7.24 (3H, m), 7.20-7.16 (2H, m), 6.11 (1H, s), 5.60 (1H, s), 4.02 (3H, s), 3.96 (3H, s), 4.92 (1H, d, *J* = 12.3 Hz), 4.82 (1H, d, *J* = 12.3 Hz), 3.06 (1H, dd, *J* = 16.7, 13.4 Hz), 2.87 (1H, ddq, *J* = 13.4, 6.7, 4.8 Hz), 2.44 (1H, dd, *J* = 16.7, 4.8 Hz), 0.99 (3H, d, *J* = 6.7 Hz); ¹³C-NMR(CDCl₃): δ 196.9, 192.3, 169.5 (2C), 164.5, 157.7, 134.6, 128.5 (2C), 128.1, 126.6 (2C), 105.9, 105.2, 97.2, 90.7, 89.4, 70.6, 56.9, 56.3, 40.0, 36.6, 14.2; EIMS: m/z calcd for C₂₃H₂₁ClO₆ [M+] 428, found 428.

The following representative compounds were prepared according to general procedure 3:
(2*S*,6'*R*)-(7-chloro-4-ethoxy-6-methoxy-benzofuran-3-on)-2-spiro-1'-(2'-methoxy-6'-methyl-cyclohex-2'-en-4'-one): m.p.: 205-206 °C; ¹H-NMR(CDCl₃): δ 6.12 (1H, s), 5.53 (1H, s), 4.19 (2H, m), 4.00 (3H, s), 3.61 (3H, s), 3.04 (1H, dd, *J* = 16.8, 13.5 Hz), 2.84 (1H, ddq., *J* = 13.5, 4.7,6.7 Hz), 2.42 (1H, dd, *J* = 16.8, 4.7 Hz), 1.52 (3H, t, *J* = 7.0 Hz), 0.96 (3H, d, *J* = 6.7 Hz); ¹³C-NMR(CDCl₃): δ 196.9, 192.2, 170.8, 169.5, 164.4, 157.2, 105.6, 104.7, 97.0, 90.6, 90.3, 65.1, 56.9, 56.6, 40.0, 36.4, 14.3, 14.2; EIMS: m/z calcd for C₁₈H₁₉ClO₆ [M+] 366, found 366.
(2*S*,6'*R*)-(7-chloro-4-benzyloxy-6-methoxy-benzofuran-3-on)-2-spiro-1'-(2'-methoxy-6'-methyl-cyclohex-2'-en-4'-one): m.p.: 237-239 °C; ¹H-NMR(CDCl₃): δ 7.49-7.46 (2H, m), 7.42-7.38 (2H, m), 7.36-7.31 (1H, m), 6.17 (1H, s), 5.55 (1H, s), 5.27 (2H, s), 3.94 (3H, s), 3.63 (3H, s), 3.05 (1H, dd, *J* = 16.6, 13.6 Hz), 2.85 (1H, m), 2.44 (1H, dd, *J* = 16.6, 4.6 Hz), 0.98 (3H, d, *J* = 6.7 Hz); ¹³C-NMR(CDCl₃): δ 196.9, 192.1, 170.8, 169.4, 164.3, 156.6, 135.4, 128.8 (2C), 128.3, 126.8 (2C), 104.8 (2C), 97.4, 91.5, 90.7, 71.1, 56.9, 56.6, 40.0, 36.5, 14.4; EIMS: m/z calcd for C₂₃H₂₁ClO₆ [M+] 428, found 428.

The following representative compounds were prepared according to the procedures supplied for each compound:
(2*S*,6'*R*)-(7-chloro-4,6-dimethoxy-benzofuran-3-on)-2-spiro-1'-(2'-methoxy-3',6'-dimethyl-cyclohex-2'-en-4'-one): To a solution of griseofulvin [Arkley et al., 1962] (514 mg, 1.5 mmol) in acetone (10 mL) was added K₂CO₃ and MeI (0.2 mL, 3.1 mmol), the mixture was then heated to 80 °C. The solution was cooled to 20 °C after 14 h and transfered to a separatory funnel with EtOAc (30 mL) and washed with aq. NH₄Cl (40 mL). The combined aqueous phases were extracted with EtOAc (3×60 mL) and the organic layer was dried (MgSO₄) then concentrated. The crude mixture was purified by column chromatography (EtOAc and heptane) to give the title compound: 29 mg (5 %): ¹H-NMR (CDCl₃): δ 6.14 (1H, s), 4.04 (3H, s), 3.98 (3H, s), 3.73 (3H, s), 3.06 (1H, dd, *J* = 16.6, 13.7 Hz), 2.78 (1H, ddq., *J* = 13.6, 4.8, 6.5 Hz), 2.47 (1H, dd, *J* = 16.6, 4.7 Hz), 1.85 (3H, s), 0.95 (3H, d, *J* = 6.7 Hz); ¹³C-NMR (CDCl₃): δ 198.0, 193.1, 169.3, 166.5, 164.4, 157.6, 125.0, 105.6, 97.2, 92.9, 89.4, 61.8, 56.9, 56.3, 40.2, 36.4, 14.2, 9.3; EIMS: m/z calcd for C₁₈H₁₉ClO₆ M+ 366, found 366.
(2*S*,2'*S*,6'*R*)-(7-chloro-4,6-dimethoxy-benzofuran-3-on)-2-spiro-1'-(2'-benzyloxy-6'-methyl-cyclohexan-4'-one): A solution of isogriseofulvin [Arkley et al., 1962] (106 mg, 0.3 mmol) in MeOH (3 mL) was cooled to -45 °C. CeCl₃ (239 mg, 1.0 mmol) was added and the solution stirred for 10 min. whereupon NaBH₄ (35 mg, 0.9 mmol) was added and the mixture was allowed to reach -35 °C. After 4 h excess reagent was quenched by addition of acetone (3 mL) over 5 min. and the mixture was allowed to warm to 20 °C. EtOAc (10 mL) was added to the solution and the mixture was washed with destilled water (20 mL). The aqueous phase was extracted with EtOAc (3×20 mL) and the organic layer was dried (MgSO₄) then concentrated and the residue was recrystallized from EtOAc and heptane, affording an intermediate, which was dissolved in EtO₂ (10 mL). Ag₂O (138 mg, 0.6 mmol) and benzyl bromide (242 mg, 0.6 mmol) was added and the mixture was heated at 50 °C for 72 h and then cooled to 20 °C. EtOAc (60 mL) was added to the solution and the mixture was filtered through silica, dried (MgSO₄) and concentrated. The crude mixture was purified by column chromatography (EtOAc and /heptane) affording the title compound, which was re-crystallized from EtOAc and heptane yielding 59 mg (45 %): m.p.: 162-163 °C; IR (KBr, cm⁻1): 1700, 1612 (C=O); ¹H-NMR (CDCl₃): δ 7.28-7.22 (3H, m), 7.14-7.11 (2H, m), 6.10 (1H, s), 4.55 (1H, d, *J* = 12.5 Hz), 4.46 (1H, d, *J* = 12.5 Hz), 4.04 (1H, dd, *J* = 12.4, 5.7 Hz), 4.03 (3H, s), 3.98 (3H, s), 3.37 (1H, dd, *J* = 14.2, 12.4 Hz), 3.15 (1H, m), 2.71 (1H, ddd, *J* = 14.3, 5.7, 2.1 Hz), 2.36 (1H, ddq., *J* = 13.4, 4.7, 6.6 Hz), 2.29 (1H, ddd, *J* = 14.7, 4.7, 2.1 Hz), 0.91 (3H, d, *J* = 6.6 Hz); ¹³C-NMR (CDCl₃): δ 206.3, 195.7, 169.0, 164.1, 157.1, 137.3, 128.3 (2C), 127.6, 127.4 (2C), 107.7, 97.0, 93.7, 89.0, 78.1, 72.1, 56.9, 56.2, 43.4, 42.8, 34.4, 14.4; EIMS: m/z calcd for C₂₃H₂₃ClO₆ M+ 430, found 430.
(2*S*,6'*R*)-(7-chloro-4,6-dimethoxy-benzofuran-3-on)-2-spiro-1'-(2'-methoxy-6'-methyl-cyclohex-3'-en-4'-oxime): To a solution of griseofulvin [1] (1.0 g, 2.84 mmol) in EtOH (120 mL) was added hydroxylamine hydrochloride (620 mg, 8.92 mmol) and sodium acetate (1.0 g, 12.19 mmol). The mixture was stirred at 80 °C for 24 hours, cooled to 0 °C, and the white precipitate was collected by filtration. CH₂Cl₂ (150 mL) was added to the mother liquor and washed with water (2×100 mL) and then brine (100 mL). The combined organic phases were dried (MgSO₄), filtrated and then concentrated to give the title compound: 988 mg (95 %): IR (KBr, cm⁻1): 1706 (C=O), 1590 (C=C), 1614 (C=N); ¹H-NMR (CDCl₃): δ 6.20 (1H, s), 6.04 (1H, s), 5.53 (1H, s), 3.95 (3H, s), 3.90 (3H, s), 3.55 (3H, s), 2.93 (1H, dd, *J* = 15.0, 13.1 Hz), 2.57-2.43 (1H, m), 2.34 (1H, dd, *J* = 15.0, 4.2 Hz), 0.88 (3H, d, *J* = 6.7 Hz); ¹³C-NMR (CDCl₃): δ 194.2, 176.4, 169.9, 155.3, 152.1, 106.0, 99.1, 97.4, 92.9, 91.6, 89.4, 57.2, 56.4, 56.2, 36.6, 35.5, 14.6; EIMS: m/z calcd for C₁₇H₁₈ClNO₆ M+ 367, found 367; Anal. Calcd for C₁₇H₁₈ClNO₆: C, 55.52; H, 4.93, found: C, 55.60; H, 4.96.

[Arkley, V., Attenburrow, J., Gregory, G. I., and Walker, T. *J*. *Chem. Soc.* **1962,** 1260-1268].

## Claims

1. The use of a compound according to the general formula (I) 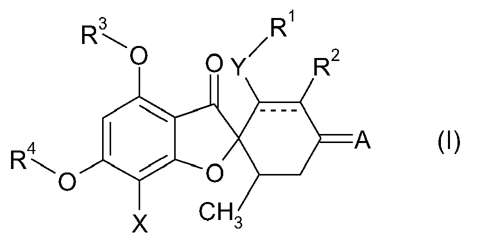 wherein the symbols have the following meanings:
A is selected from: =O, =NOR⁵, and =N-NR⁶R⁷, and =NR⁸;
X is selected from: H, halogen and pseudohalogen;
Y is selected from: -CH₂-, -O-, -S- and -NH-;
wherein the dotted line denotes a double bond which may optionally be present;
R¹ is selected from: acyclic and cyclic, linear and branched alkyl, alkenyl and alkynyl groups having 4 to 20 carbon atoms, wherein the named groups can be unsubstituted or substituted or carry one or more substituents from the group halogen, pseudohalogen, -C(O)OH, -NO₂, NH₂, -OH and -O(C₁-C₄)alkyl; linear or branched acyclic alkyl groups having 1 to 20 carbon atoms and carrying in their chain one or more non-adjacent atoms from the group O and S; and aralkyl groups having a linear or branched acyclic alkyl group with 1 to 20 carbon atoms and an aromatic group having 5 to 10 carbon atoms, in which the aromatic group optionally contains one or more heteroatoms selected from O, S or N in its cycle;
R² is selected from: H; acyclic and cyclic, linear and branched alkyl, alkenyl and alkynyl groups having 1 to 20 carbon atoms, wherein the named groups can be unsubstituted or substituted or carry one or more substituents from the group halogen, pseudohalogen, -C(O)OH, -NO₂, NH₂, -OH and -O(C₁-C₄)alkyl; linear or branched acyclic alkyl groups having 1 to 20 carbon atoms and carrying in their chain one or more non-adjacent atoms from the group O and S; and aralkyl groups having a linear or branched acyclic alkyl group with 1 to 20 carbon atoms and an aromatic group having 5 to 10 carbon atoms, in which the aromatic group optionally contains one or more heteroatoms selected from O, S or N in its cycle;
R³ is selected from: H; acyclic and cyclic, linear and branched alkyl, alkenyl and alkynyl groups having 1 to 10 carbon atoms; and aralkyl groups having a linear or branched acyclic alkyl group with 1 to 10 carbon atoms and an aromatic group having 5 or 6 carbon atoms;
R⁴ independently has the same meaning as R³;
R⁵ is H or a linear or branched alkyl group having 1 to 4 carbon atoms or an acyl group having 1 to 4 carbon atoms;
R⁶ is H or a linear or branched alkyl group having 1 to 4 carbon atoms;
R⁷ independently has the same meaning as R⁶;
R⁸ independently has the same meaning as R⁶;
and/ or a pharmaceutically acceptable salt thereof,
for the manufacture of a pharmaceutical composition for the treatment of cancer.

2. The use according to claim 1, wherein
A is selected from: =O, =NOR⁵;
X is selected from: H, halogen and pseudohalogen;
Y is selected from: -CH₂-, -O-;
wherein the dotted line denotes a double bond which may optionally be present;
R¹ is selected from: acyclic and cyclic, linear and branched alkyl, alkenyl and alkynyl groups having 4 to 15 carbon atoms, wherein the named groups can be unsubstituted or substituted or carry one or more substituents from the group halogen, pseudohalogen, -C(O)OH, -NO₂, NH₂, -OH and -O(C₁-C₄)alkyl; linear or branched alkylenoxy groups having 1 to 20 carbon atoms; and aralkyl groups having a linear or branched acyclic alkyl group with 1 to 10 carbon atoms and an aromatic group having 5 or 6 carbon atoms, in which the aromatic group optionally contains one or more heteroatoms selected from O, S or N in its cycle;
R² is selected from: H; acyclic and cyclic, linear and branched alkyl, alkenyl and alkynyl groups having 1 to 15 carbon atoms, wherein the named groups can be unsubstituted or substituted or carry one or more substituents from the group halogen, pseudohalogen, -C(O)OH, -NO₂, NH₂, -OH and -O(C₁-C₄)alkyl; linear or branched alkylenoxy groups having 1 to 20 carbon atoms; and aralkyl groups having a linear or branched acyclic alkyl group with 1 to 10 carbon atoms and an aromatic group having 5 or 6 carbon atoms, in which the aromatic group optionally contains one or more heteroatoms selected from O, S or N in its cycle;;
R³ is selected from: H; acyclic and cyclic, linear and branched alkyl, alkenyl and alkynyl groups having 1 to 6 carbon atoms; and aralkyl groups having a linear or branched acyclic alkyl group with 1 to 6 carbon atoms and an aromatic group having 5 or 6 carbon atoms;
R⁴ independently has the same meaning as R³;
R⁵ is H or a linear or branched alkyl group having 1 to 4 carbon atoms or an acyl group having 1 to 4 carbon atoms;

3. The use according to claim 1 or 2, wherein
A is selected from: =O, =NOR⁵;
X is selected from: H, halogen and pseudohalogen;
Y is selected from: -CH₂-, -O-;
wherein the dotted line denotes a double bond which may optionally be present;
R¹is selected from: acyclic and cyclic, linear and branched alkyl, alkenyl and alkynyl groups having 4 to 10 carbon atoms, wherein the named groups can be unsubstituted or substituted or carry one or more substituents from the group halogen, pseudohalogen, -C(O)OH, -OH and -O(C₁-C₄)alkyl; ethylenoxy groups having 1 to 20 carbon atoms and propylenoxy groups having 1 to 20 carbon atoms; and aralkyl groups having a linear or branched acyclic alkyl group with 1 to 6 carbon atoms and an aromatic group having 5 or 6 carbon atoms, in which the aromatic group optionally contains one or more heteroatoms selected from O, S or N in its cycle;
R² is selected from: H; acyclic and cyclic, linear and branched alkyl, alkenyl and alkynyl groups having 1 to 10 carbon atoms, wherein the named groups can be unsubstituted or substituted or carry one or more substituents from the group halogen, pseudohalogen, -C(O)OH, -OH and -O(C₁-C₄)alkyl; ethylenoxy groups having 1 to 20 carbon atoms and propylenoxy groups having 1 to 20 carbon atoms; and aralkyl groups having a linear or branched acyclic alkyl group with 1 to 6 carbon atoms and an aromatic group having 5 or 6 carbon atoms, in which the aromatic group optionally contains one or more heteroatoms selected from O, S or N in its cycle;
R³ is selected from: H; acyclic and cyclic, linear and branched alkyl, alkenyl and alkynyl groups having 1 to 6 carbon atoms; and aralkyl groups having a linear or branched acyclic alkyl group with 1 to 6 carbon atoms and an aromatic group having 5 or 6 carbon atoms;
R⁴ independently has the same meaning as R³;
R⁵ is H or a linear or branched alkyl group having 1 to 4 carbon atoms or an acyl group having 1 to 4 carbon atoms;

4. The use according to any of claims 1 to 3, wherein
A is selected from: =O, =NOR⁵;
X is selected from: H, halogen;
Y is selected from: -O-;
wherein the dotted line denotes a double bond which may optionally be present;
R¹ is selected from: acyclic and cyclic, linear and branchched alkyl, alkenyl and alkynyl groups having 5 to 8 carbon atoms, wherein the named groups can be unsubstituted or substituted or carry one or more substituents from the group halogen, pseudohalogen, -C(O)OH, -OH and -O(C₁-C₄)alkyl; ethylenoxy groups having 1 to 10 carbon atoms and propylenoxy groups having 1 to 10 carbon atoms; and aralkyl groups having a linear or branched acyclic alkyl group with 1 to 4 carbon atoms and an aromatic group having 5 or 6 carbon atoms, in which the aromatic group optionally contains one or more heteroatoms selected from O, S or N in its cycle;
R² is selected from: H; acyclic and cyclic, linear and branchched alkyl, alkenyl and alkynyl groups having 1 to 8 carbon atoms, wherein the named groups can be unsubstituted or substituted or carry one or more substituents from the group halogen, pseudohalogen, -C(O)OH, -OH and -O(C₁-C₄)alkyl; ethylenoxy groups having 1 to 10 carbon atoms and propylenoxy groups having 1 to 10 carbon atoms; and aralkyl groups having a linear or branched acyclic alkyl group with 1 to 4 carbon atoms and an aromatic group having 5 or 6 carbon atoms, in which the aromatic group optionally contains one or more heteroatoms selected from O, S or N in its cycle;
R³ is selected from: H; acyclic and cyclic, linear and branched alkyl, alkenyl and alkynyl groups having 1 to 4 carbon atoms; and aralkyl groups having a linear or branched acyclic alkyl group with 1 to 4 carbon atoms and an aromatic group having 5 or 6 carbon atoms;
R⁴ independently has the same meaning as R³;
R⁵ is H or a linear or branched alkyl group having 1 to 4 carbon atoms or an acyl group having 1 to 4 carbon atoms;

5. The use according to any of the claims 1 to 4, wherein the cancer is selected from the group human malignancy, preferably solid neoplasia or haematological malignancy.

6. The use according to claim 5, wherein said cancer is selected from brain cancer, head-and neck cancer, breast cancer, esophageal cancer, gastric cancer, colon cancer, liver cancer, lung cancer, renal cancer, pancreas cancer, biliary tract cancer, prostate cancer, skin cancer, melanoma, ovarian cancer, cervical cancer, sarcoma, bone and soft tissue sarcomas, leukemia, multiple myeloma and lymphoma, including both Hodgkin and Non-Hodgkin lymphomas.

7. The use of a compound as defined in any of the claims 1 to 4, or a pharmaceutically acceptable salt thereof, for the manufacture of a pharmaceutical composition for the inhibition of centrosome clustering.

8. The use of a compound as defined in any of the claims 1 to 4, or a pharmaceutically acceptable salt thereof, for the manufacture of a pharmaceutical composition to induce multipolar mitoses in cells with supernumerary centrosomes.

9. The use of a compound as defined in any of the claims 1 to 4, or a pharmaceutically acceptable salt thereof, for the manufacture of a pharmaceutical composition to induce apoptosis in cells.

10. A compound as defined in any of the claims 1 to 4, or a pharmaceutically acceptable salt thereof, for use as a medicament.

11. A compound as defined in any of the claims 1 to 4, or a pharmaceutically acceptable salt thereof.

12. A method for treating cancer in a patient, comprising administering to a patient suffering from said disease in a therapeutically effective amount a compound as defined in any of claims 1 to 4.

13. A pharmaceutical composition for the treatment of cancer, comprising a therapeutically effective amount of a compound as defined in any of claims 1 to 4, and optionally carriers and additives.
